# EUROPEAN PATENT SPECIFICATION

(11) **EP 2 243 508 B1**
(45) Date of publication and mention of the grant of the patent: **30.03.2016**
(21) Application number: 10007572.0
(22) Date of filing: 05.04.2005
(51) Int. Cl.: A61M 25/10

(54) **Methods for modifying a balloon of a catheter assembly**
Verfahren zur Modifizierung eines Ballons einer Kathetereinheit
Procédés de modification de ballon d'un ensemble de cathéter

(30) Priority: 07.04.2004 US 820316
(43) Date of publication of application: 27.10.2010
(62) Divisional of application: 05731961.8
(73) Proprietor: Abbott Cardiovascular Systems Inc., Santa Clara, CA 95054 (US)
(72) Inventor: Hossainy, Syed F.A., Fremont California 94555 (US); Sridharan, Srinivasan, Bel Air Maryland 21015 (US)
(74) Representative: Boult Wade Tennant

(56) References cited:
- WO-A-96/14895
- GB-A- 863 048
- US-A- 5 893 840
- US-A- 6 010 480
- US-A1- 2002 037 358
- US-A1- 2002 187 288
- US-A1- 2003 180 488
- US-B1- 6 544 223
- ANONYMOUS: 'Vacuum drying oven', [Online] 01 January 2009, XP055148978 Retrieved from the Internet: <URL:http://www.atmosafe.net/en/pdf/glossar y/thermal-ovendrying-oven/vacuum.html?filen ame=Vacuum.pdf> [retrieved on 2014-10-27]
- ANONYMOUS: 'Natural convection/forced air circulation', [Online] 01 January 2009, XP055149029 Retrieved from the Internet: <URL:http://www.atmosafe.net/en/pdf/glossar y/thermal-ovendrying-oven/natural-convectio nforced-air-circulation.html?filename=Natur al convection/forced air circulation.pdf> [retrieved on 2014-10-27]

## Description

### BACKGROUND

### Field of the Invention

This invention is directed to methods for modifying a balloon of a catheter assembly.

### Description of the State of the Art

Balloon catheters are used for a variety of different procedures, such as percutaneous transluminal coronary angioplasty (PTCA) and stent delivery. In PTCA, a catheter assembly having a balloon, integrated at an end segment of the catheter, is introduced percutaneously into the cardiovascular system of a patient via the brachial or femoral artery. The catheter is advanced through the coronary vasculature until the balloon portion is positioned across the occlusive lesion. Once in position across the lesion, the balloon is inflated to a predetermined size to radially compress against the atherosclerotic plaque of the lesion to remodel the lumen wall. The balloon is then deflated to a smaller profile to allow the catheter to be withdrawn from the patients' vasculature.

In addition to remodeling of the vessel wall, balloons have been used to deliver a therapeutic substance at the occlusion site. Balloons having a porous wall membrane can be inflated with a fluid carrier including a therapeutic substance. Upon inflation of the balloon, the therapeutic fluid is expelled out from the porous wall membrane. Alternatively, a balloon can be coated with a therapeutic substance for delivery of the substance at the treatment site. One of the problems associated with a porous balloon membrane is the trauma that may be inflicted on the vessel walls caused by the ejection of the fluid out from the porous balloon membrane. If the fluid carrier is expelled at too high a velocity, it can cause damage to the vessel wall, despite its medicinal properties. This has been referred to as the "jetting effect" To counterbalance the "jetting effect," the pores have been reduced in size to muffle the velocity of the therapeutic fluid. Minimizing the pore size has provided manufacturing challenges. Simple coating of balloons with a therapeutic substance has provided an inadequate platform for the local delivery of a drug to the occluded site. By the time the balloon reaches the intended site, most, if not all, of the drug will have washed away off the balloon. Accordingly, there is a need to provide an effective means of delivering a drug from a balloon.

Nonetheless, there is described in US 5,893,840 a balloon catheter which includes body affecting chemicals within microcapsules on the exterior of the balloon either alone or with a stent. The coating releases from the balloon when the balloon is inflated into contact with the lumen to be treated. The device provides placement of the dosage required at the location in need of treatment. A sheath may be employed over the balloon and microcapsules to prevent the microcapsules from being rubbed off during delivery through a body lumen.

US 2002/0187288 describes a medical device having a body formed of melt process extruded, porous silicone polyurethane material. The silicone polyurethane is combined with a porogen and then melt process extruded into a desired shape such as a tubular body. The porogen is then extracted from the extrudate, to form the extruded, melt processed, porous silicone polyurethane tubular body. The medical device may comprise a stent cover, vascular graft, or catheter balloon, formed of the silicone polyurethane and has biostability, strength and flexibility.

US 2003/0180488 describes a catheter balloon formed of a polymeric material such as expanded polytetrafluoroethylene (ePTFE) bonded to a second layer formed of a low tensile set polymer and/or impregnated with a low tensile set polymer. The low tensile set polymer is an elastomer selected from the group consisting of a silicone-polyurethane copolymer and a diene polymer. The low tensile set polymer has high strength, low modulus, high elongation, and low tensile set, to provide improved balloon performance. The diene or silicone-polyurethane has a low tensile set, which facilitates deflation of the balloon to a low profile deflated configuration.

US 6,544,223 describes a drug delivery device for delivering therapeutic agents and a method of making such a device. The device includes an inflatable balloon having a plurality of holes formed in the wall of the balloon. A microporous coating covers a portion of the outer surface of the wall of the balloon. The thickness of the coating and the size of the micropores can permit controlled delivery of a substance from the micropores of the coating and into the tissue of a patient's lumen.

For stent delivery, a stent can be securely crimped on the balloon. The balloon can be the same balloon used for the remodeling of the vessel wall or a second stent delivery balloon can be introduced into the patient. At the designated site, the stent is deployed by the balloon, and then the balloon is deflated and withdrawn from the bore of the stent, leaving the stent to maintain vascular patentcy and optionally to deliver a therapeutic substance. A stent can be modified to deliver a therapeutic substance by a polymeric coating. Briefly, a polymer dissolved in a solvent and a therapeutic agent added thereto can be applied to the surface of a stent. The solvent is evaporated, leaving a polymeric coating, impregnated with a therapeutic substance, on the stent surface. A polymeric coating can increase the coefficient of friction between the stent and the balloon of a catheter assembly on which the stent is crimped for delivery. Additionally, some polymers have a "sticky" or "tacky" consistency. If the polymeric material either increases the coefficient of friction or adherers to the catheter balloon, the effective release of the stent from the balloon after deflation can be compromised. If the stent coating adheres to the balloon, the coating, or parts thereof, can be pulled off the stent during the process of deflation and withdrawal of the balloon following the placement of the stent. Adhesive, polymeric stent coatings can also experience extensive balloon sheer damage post-deployment, which could result in a thrombogenic stent surface and embolic debris. The stent coating can stretch when the balloon is expanded and may delaminate as a result of such shear stress. Accordingly, there is a need to eliminate or minimize damage caused to a coating of a stent by the delivery balloon. The embodiments of the present invention provide for methods to modify the balloon to achieve this as well as other results.

### SUMMARY

In accordance with the present invention there is provided a method of modifying a balloon of a catheter assembly as recited in appended Claim 1. In some embodiments, the inflated state is greater than a range of an intended expanded configuration of the balloon. In other embodiments, the inflated state is less than a range of an intended expanded configuration of the balloon.

### BRIEF DESCRIPTION OF THE DRAWINGS

FIG. 1 illustrates a balloon integrated on a catheter assembly; the balloon is illustrated in a collapsed configuration, an under inflated state, an intended inflated state, and a hyper or over inflated state.
FIGs. 2 and 3 are SEM microphotographs showing ePTFE balloons after the immersion of the balloons in a solution of EVEROLIMUS.
FIG. 4 is an optical microphotograph comparison of dyed and non-dyed ePTFE balloons.

### DETAILED DESCRIPTION

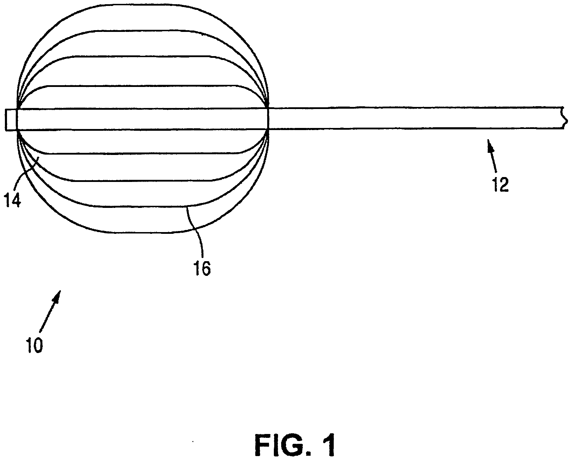

FIG. 1 illustrates a balloon 10 incorporated at an end segment of a catheter 12. The balloon 10 is intended to include any type of enclosed member such as an elastic type member that is selectively inflatable to dilate from a collapsed configuration to a desired and controlled expanded configuration. The balloon 10 should also be capable of being deflated to a reduced profile or back to its original collapsed configuration. The balloon 10 can be made of any suitable type of material and can be of any thickness so long as the ability to modify the balloon and optimum performance capabilities of the balloon are not adversely compromised. Modification of the balloon will be discussed in detail below. Performance properties include high burst strength, good flexibility, high resistance to fatigue, an ability to fold, and an ability to cross and re-cross a desired region of treatment or an occluded region in a body lumen, and a low susceptibility to defects caused by handling, among other possibilities.

A material of the balloon is porous. Porous is intended to include not only cavities or surface depots created by a manufacturing process (e.g., laser drilling or etching) but also inherent properties of or spaces within the lattice structure of a polymeric material. Examples of materials that can be used include poly(tetrafluoroethylene)(PTFE), expanded poly(tetrafluoroethylene) (ePTFE), or expanded poly(ethylene). One variety of expanded poly(ethylene) that can be used includes expanded ultra-high molecular weight polyethylene, having a molecular weight between about 500,000 and about 10,000,000 Daltons. Examples of some other porous materials that can be used to make a balloon include expanded poly(trifluoro ethylene) (e.g., EASYSTREET balloon available from Guidant Corp.), poly(urethanes), poly(amides), poly(esters), and poly(ethylenes), including an ultra high molecular weight (polyethylene). Examples of poly(urethanes) include poly(ester urethanes), poly(ether urethanes), poly(silicone urethanes), and poly(carbonate urethanes). In particular, poly(urethane) products such as PELLETHANE or TECOTHANE can be used. Examples of some poly(esters) that can be used include poly(ethylene terephthalate) and poly(butylene terephthalate). Examples of some poly(amides) that can be used include NYLON and PEBAX. PELLETHANE is a trade name of a family of thermoplastic polyurethane elastomers having ether, ester, or caprolactone fragments. PELLETHAN products are available from Dow Chemical Co. of Midland, Michigan. TECOTHANE is a trade name of a family of thermoplastic aromatic poly(ether urethanes). TECOTHANE products are available from Thermedics Polymer Products Co. of Wilmington, Massachusetts. NYLON is a trade name of a family of poly(amides). NYLON products are available from E.I. DuPont deNemours Co. of Wilmington, Delaware. PEBAX is a trade name of a family of poly(ether)-block-poly(amide) copolymers. PEBAX products are available from Atofina Chemicals, Inc. of Philadelphia, Pennsylvania.

The balloon comprises two layers - an inner layer made of a non-porous material and an outer layer made of a porous material, such as cross-linked hydrogel made of a copolymer of poly(ethylene glycol) and a polymeric acid such as poly(lactic acid), poly(glycolic acid), poty(lactic acid-co-glycolie acid) and mixtures thereof. The outer cross-linked hydrogel has pores that can be filled with a drug or other types of agents.

FIG. 1 illustrates the balloon 10 in its collapsed configuration 14 as well as its intended deployment or expanded configuration 16. Collapsed configuration 14 is the state of complete deflation such as when no gas or fluid is introduced into the balloon 10. A balloon is inserted into a patient and maneuvered to the designated area of treatment in its collapsed configuration. The intended expanded configuration is defined as the inflation of a balloon to a diameter or size within the range of its intended use or design. The intended expanded configuration is provided by the manufacturer of the balloon (or can be determined by one having ordinary skill in the art) and is intended to include the range of diameters of use or the range of pressures to be applied for the planned performance of the balloon. Under inflation is defined as any diameter between the collapsed configuration and the intended expanded configuration. Over or hyperinflation is defined as any diameter above the intended expanded configuration but less than a diameter or size in which the balloon will be damaged or no longer suitable for its intended use. The term "inflation," "inflated," "inflated state," or "expanded" is to include, unless otherwise specified, under inflation, intended expanded configuration as well as hyperinflation.

The balloon can be modified with one or a combination of a drug or therapeutic substance, a polymer, or a blocking agent. Modification is intended to include deposition of the substance within the balloon wall membrane. In other words, the substance penetrates within the membrane from which the balloon is made. The substance, such as the blocking agent, can be a fluid form by itself or when mixed or dissolved in a solvent. Modification can be achieved by, for example, spraying or brushing a modifying substance on the balloon or, preferably, dipping the balloon in the solution of the substance. The substance can be dissolved, saturated or supersaturated in a solvent. The duration of exposure needs to be long enough so as to allow penetration into the pores or the lattice structure of the polymer. In some embodiments, the balloon is first inflated and then the modifying substance is applied to the balloon. For example, the balloon is first inflated and then immersed into a modifying substance or sprayed with the modifying substance. Alternatively, a modifying substance is applied first and then the balloon is inflated. For example, the balloon is immersed in a solvent solution and then the balloon is inflated. In some embodiments, the state of inflation should be maintained during the modification process. For example, if the balloon is hyper-inflated, during the course of the process, the balloon should remain hyper-inflated with no or only a negligible fluctuation in the balloon diameter or pressure applied in the balloon. In other embodiments, the state of inflation can be gradually increased or reduced during the modification process. For example, the state of inflation can be reduced from a hyper-inflated state to an under inflated state during the modification process.

When a wet (e.g., solvent) application is employed, in some embodiments the state of inflation of the balloon is maintained during the drying process. That is, the state of inflation during the application of a solvent and a modifying agent is generally the same as the state of inflation during the evaporation of the solvent. In other embodiments, prior to or during the drying process, the state of inflation of the balloon can be modified to a different state. For example, the balloon can be modified at a hyper-inflated state and dried in its intended expanded configuration or an under inflated state; the balloon can be modified in its intended expanded configuration and can be dried in an under inflated state or in a hyper-inflated state; or the balloon can be modified in an under inflated state and dried in the intended expanded configuration or a hyper-inflated configuration. In some embodiments, the drying can be conducted in a deflated state such that prior to or during the drying process, the pressure applied to the balloon can be reduced negligibly or significantly so as to collapse the pores. Drying or evaporation of the solvent can be expedited with the application of heat.

During the application of the modifying substance and/or during the drying process of a wet substance, in some embodiments, the balloon can be pulsed. Pulsing or pulsating is defined as increasing and/or decreasing the diameter or size of the balloon for one cycle or more. For example, the balloon can be pulsed in an under inflated state such that the pulsing action does not inflate the balloon to the intended expanded diameter state. Alternatively, the balloon can be pulsed from an under inflated state to the intended expanded state and back to the under inflated state during the coating procedure. This can be repeated more than once. In another example, the balloon can be pulsed from an intended expansion state to a hyper-inflated state. In another example, the balloon can be pulsed from the hyper-inflated state to the intended expanded configuration for more than one time. The pulsing action can carry into the drying stage. In some embodiments, the pulsing is done only in the drying stage and not the modification state.

A gas, such as air or an inert gas (e.g. argon or nitrogen) is applied to the balloon contemporaneously with the application of the modifying substance. The temperature of the gas can depend on the volatility of the fluid or solvent carrier. A "volatile solvent" means a solvent that has a vapor pressure greater than 17.54 Torr (2.34 kPa) at ambient temperature, and a "non-volatile solvent" means a solvent that has a vapor pressure of less than or equal to 17.54 Torr (2.34 kPa) at ambient temperature. A warm gas may be particularly suitable when the solvent employed in the composition is a non-volatile solvent (e.g., dimethylsulfoxide (DMSO), dimethylformamide (DMF), and dimethylacetamide (DMAC)). The temperature of the warm gas is from about 25°C to about 200°C, more narrowly from about 40°C to about 90°C. In an alternative example not forming part of the invention, a gas can be directed onto the balloon to inhibit evaporation of the solvent from the composition. Inhibition of evaporation of a solvent may be useful if the solvent is extremely volatile because the solvent may evaporate too quickly and not be capable of penetrating into the balloon membrane. In order to reduce the rate of evaporation of a solvent, a cool gas with a temperature of about less than 25°C can be used. The temperature of the gas can be, for example, significantly less than the boiling temperature of the solvent. The flow speed of the gas is from about 300 feet/minute (91.5 meters/minute) to about 10,000 feet/minute (3047.85 meters/minute), more narrowly about 2500 feet/minute (761.96 meters/minute) to about 6000 feet/minute (1828.71 meters/minute). The gas is applied for about 1 second to about 100 seconds, more narrowly for about 2 seconds to about 20 seconds. The application of the modifying substance and gas are applied in any number of cycles until the desired amount of substance is retained by the balloon. In some applications, the balloon can be rotated during the application of the substance and/or the drying stage.

Examples of drugs or therapeutic substances that can be used include any substance capable of having a therapeutic, prophylactic or diagnostic effect on a patient. Examples of therapeutic substances that can be used include antiproliferative substances such as actinomycin D, or derivatives and analogs thereof (manufactured by Sigma-Aldrich of Milwaukee, Wisconsin, or COSMEGEN available from Merck). Synonyms of actinomycin D include dactinomycin, actinomycin IV, actinomycin I₁, actinomycin X₁, and actinomycin C₁. The active agent can also fall under the genus of antineoplastic, anti-inflammatory, antiplatelet, anticoagulant, antifibrin, antithrombin, antimitotic, antibiotic, antiallergic and antioxidant substances. Examples of such antineoplastics and/or antimitotics include paclitaxel (e.g. TAXOL^{®} by Bristol-Myers Squibb Co., Stamford, Conn.), docetaxel (e.g. Taxotere^{®}, from Aventis S.A., Frankfurt, Germany) methotrexate, azathioprine, vincristine, vinblastine, fluorouracil, doxorubicin hydrochloride (e.g. Adriamycin^{®} from Pharmacia & Upjohn, Peapack N.J.), and mitomycin (e.g. Mutamycin^{®} from Bristol-Myers Squibb Co., Stamford, Conn.). Examples of such antiplatelets, anticoagulants, antifibrin, and antithrombins include sodium heparin, low molecular weight heparins, heparinoids, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antagonist antibody, recombinant hirudin, and thrombin inhibitors such as ANGIOMAX (Biogen, Inc., Cambridge, Mass.). Examples of such cytostatic or antiproliferative agents include angiopeptin, angiotensin converting enzyme inhibitors such as captopril (e.g. Capoten^{®} and Capozide^{®} from Bristol-Myers Squibb Co., Stamford, Conn.), cilazapril or lisinopril (e.g. Pimivil^{®} and Prinzide^{®} from Merck & Co., Inc., Whitehouse Station, NJ); calcium channel blockers (such as nifedipine), colchicine, fibroblast growth factor (FGF) antagonists, fish oil (omega 3-fatty acid), histamine antagonists, lovastatin (an inhibitor of HMG-CoA reductase, a cholesterol lowering drug, brand name Mevacor^{®} from Merck & Co., Inc., Whitehouse Station, NJ), monoclonal antibodies (such as those specific for Platelet-Derived Growth Factor (PDGF) receptors), nitroprusside, phosphodiesterase inhibitors, prostaglandin inhibitors, suramin, serotonin blockers, steroids, thioprotease inhibitors, triazolopyrimidine (a PDGF antagonist), and nitric oxide. An example of an antiallergic agent is permirolast potassium. Other therapeutic substances or agents which may be appropriate include alpha-interferon, genetically engineered epithelial cells, tacrolimus, dexamethasone, and rapamycin and structural derivatives or functional analogs thereof, such as 40-O-(2-hydroxy)ethyl-rapamycin (known by the trade name of EVEROLIMUS available from Novartis), 40-O-(3-hydroxy)propyl-rapamycin, 40-O-[2-(2-hydroxy)ethoxy]ethyl-rapamycin, and 40-O-tetrazole-rapamycin.

A blocking agent is intended to reduce adhesion and/or friction between a polymer coated stent and the balloon so as to minimize balloon damage to the polymeric coating of a stent. In some embodiments, a blocking agent is intended to have a reverse effect, i.e., to increase adhesion and/or friction between a polymer coated stent or a bare stent and the balloon. This may be useful if the bare stent or the polymer used to make the coating is too slippery so as to not allow the stent to remain adequately crimped on the balloon. Examples of blocking agents that can be used include sucrose, poly(ethylene glycol)(PEG), poly(ethylene oxide)(PEO), solvent-soluble fluorinated polymers, block copolymers of bioabsorbable polymers with perfluorinated end chains, SILWET surfactants (available from Union Carbide Corp.), FLUORAD surfactants (available from 3M Co.), non-ionic surfactants having alkyl, perfluorinated, or silicone chains, fatty alcohols, waxes, fatty acid salts, mono-, di-, and triglycerides, cholesterol, lecithin, dextran, dextrin, esters and ethers of cellulose, e.g., carboxymethyl cellulose and cellulose acetate, cellulosics, maltose, glucose, mannose, trehalose, sugars, poly(vinyl alcohol)(PVA), poly(2-hydroxyethyl methacrylate), poly(N-vinyl-pyrrolidone)(PVP), silicone oil, paraffins, paraffin oil, and inorganic powders, such as talcum powder, calcium salt powder, and magnesium salt powder. Other carbohydrates such as starches and dextrose can also serve as a blocking agent. Hyaluronic acid can also be used to reduce friction and/or adhesion. In some embodiments, the blocking agent can simultaneously serve as a drug. Examples of such dual-function blocking agents include steroids, clobetasol, estradiol, dexamethasone, paclitaxel, rapamycin, (available from Wyeth Pharmaceuticals of Madison, New Jersey, under the name sirolimus), and structural derivative or functional analogs of rapamycin, such as 40-O-(2-hydroxy)ethyl-rapamycin (known by the trade name of EVEROLIMUS available from Novartis), 40-O-(3-hydroxy)propyl-rapamycin, 40-O-[2-(2-hydroxy)ethoxylethyl-rapamycin, and 40-O-tetrazole-rapamycin, and drugs with an octanol/water partition coefficient greater than 100.

Polymers that are hydrophilic or hydrophobic can be used to modify the balloon. These polymers can, in some embodiments, be combined with a drug and/or blocking agent. Examples of polymers that can be used include poly(ethylene-co-vinyl alcohol) (EVAL), poly(hydroxyvalerate), poly(L-lactic acid), polycaprolactone, poly(lactide-co-glycolide), poly(hydroxybutyrate), poly(hydroxybutyrate-co-valerate), polydioxanone, polyorthoester, polyanhydride, poly(glycolic acid), poly(D,L-lactic acid), poly(glycolic acid-co-trimethylene carbonate), polyphosphoester, polyphosphoester urethane; poly(amino acids), cyanoacrylates, poly(trimethylene carbonate), poly(iminocarbonate), co-poly(ether-esters) (e.g. PEO/PLA), polyalkylene oxalates, polyphosphazenes, biomolecules (such as fibrin, fibrinogen, cellulose, starch, collagen and hyaluronic acid), polyurethanes, silicones, polyesters, polyolefins, polyisobutylene and ethylene-alphaolefin copolymers, acrylic polymers and copolymers, vinyl halide polymers and copolymers (such as polyvinyl chloride), polyvinyl ethers (such as polyvinyl methyl ether), polyvinylidene halides (such as polyvinylidene fluoride and polyvinylidene chloride), polyacrylonitrile, polyvinyl ketones, polyvinyl aromatics (such as polystyrene), polyvinyl esters (such as polyvinyl acetate), copolymers of vinyl monomers with each other and olefins (such as ethylene-methyl methacrylate copolymers, acrylonitrile-styrene copolymers, ABS resins, and ethylene-vinyl acetate copolymers), polyamides (such as Nylon 66 and polycaprolactam), alkyd resins, polycarbonates, polyoxymethylenes, polyimides, polyethers, epoxy resins, rayon, rayon-triacetate, cellulose, cellulose acetate, cellulose butyrate, cellulose acetate butyrate, cellophane, cellulose nitrate, cellulose propionate, cellulose ethers, and carboxymethyl cellulose.

In some embodiments, the balloon can be modified with a low adhesion polymer to prevent damage to a polymer coated stent. Low adhesion polymers can be fully or partially fluorinated or non-fluorinated. Examples of low adhesion fluorinated polymers that can be used include poly(tetrafluoro ethylene) (PTFE), poly(vinylidene fluoride)(PVDF), and poly(vinylidene fluoride-co-hexafluoropropene) (PVDF-HFP). Various brands of PTFE can be used, including any product of the TEFLON family available from E. I. DuPont de Nemours of Wilmington, Delaware. Various brands of PVDF-HFP known as the SOLEF family of products, available from Solvay Fluoropolymers, Inc. of Houston, Texas, can be used, for example, SOLEF 21508 having about 85 mass % of vinylidene fluoride-derived units and about 15 mass % of hexafluoro propene-derived units. PVDF-HFP is also available from Atofina Chemicals of Philadelphia, Pennsylvania, under the trade name KYNAR. Examples of low adhesion non-fluorinated polymers that can be used include poly(n-butyl methacrylate)(PBMA), poly(methyl methacrylate)(PMMA), poly(ethyl methacrylate)(PENIA), polycarbonate, polystyrene and poly(butyleneterephthalate-co-ethylene glycol) (PBT-PEG). In some embodiments a family of PBT-PEG known as POLYACTIVE can be used. POLYACTIVE is a trade name of a PBT-PEG group of products and is available from IsoTis Corp. of Holland. In various brands of POLYACTIVE, the ratio between the units derived from ethylene glycol and the units derived from butylene terephthalate can be between about 0.67:1 and about 9:1. The molecular weight of the units derived from ethylene glycol can be between about 300 and about 4,000 Daltons. Alternatively, in some embodiments, the balloon can be modified with a high adhesion polymer to allow a bare stent or a polymer having slippery characteristics to remain on the balloon during delivery and expansion of the stent.

A variety of solvents, such as isopropyl alcohol, can be used for making the solution to be applied to the balloon, taking into account both the solubility of a drug, polymer and/or blocking agent and the ability of the solvent to wet the pores and penetrate into the balloon material. For balloons made of poly(tetrafluoroethylene), for example, preferable solvents include acetonitrile, acetone or isopropanol.

A drug or cocktail combination of drugs can be delivered to a patient using a dual mode delivery, i.e., both via a balloon and a stent. The dual mode of the delivery is believed to be particularly beneficial for the treatment of multimodal pathologies such as restenosis. One beneficial effect that can be provided by the dual mode delivery is believed to be an ability to achieve better inhibition of restenosis. The term "inhibition" refers to reduction, elimination, prevention, or treatment of restenosis, and includes delaying the onset of the cellular activity leading to the condition. The first mode of delivery provides for delivery of a drug via the balloon of the delivery catheter, and the second mode of delivery provides for the local drug delivery via a coated stent after the stent has been positioned in place and deployed. The dual mode delivery may produce a synergistic beneficial therapeutic effect compared with the effect produced by drug delivery using either mode of delivery alone. The balloon can also provide for a quick burst of a drug followed by a prolonged local administration of the same drug or another drug by a stent. When the balloon expands, the pores can open up, releasing the embedded drug. The balloon can deliver a drug immediately before the time of deployment or implantation of the stent; substantially contemporaneously with the deployment or implantation of the stent; and/or immediately after the time of deployment or implantation of the stent.

### Example 1 - Simulated Experiment

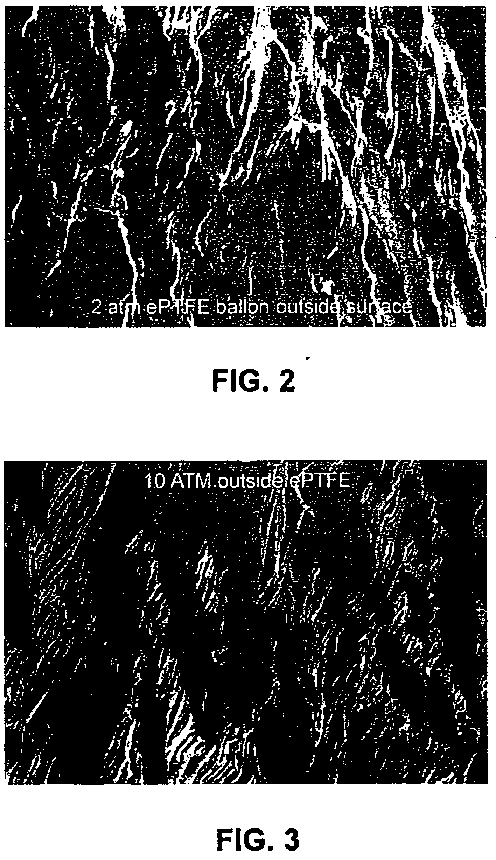

A solution containing about 2 mass % EVEROLIMUS, and the balance, acetonitrile, was prepared. One drop of a blue azo dye was added for contrast. Two balloon sub-assemblies, each containing an ePTFE balloon were made. Both sub-assemblies were immersed in the EVEROLIMUS solution for about 30 seconds and then removed and visually inspected. Very minimal blue staining was observed in each case, indicating that not more than a very small, negligible, amount of EVEROLIMUS was impregnated into the balloon membranes. The very insignificant penetration of EVEROLIMUS into the balloon can be explained by the fact that the ePTFE balloon in the uninflated state contained very few pores, as shown by FIG. 2.

The balloon of the first sub-assembly was then inflated using saline solution to about 8 atmospheres and immersed into the EVEROLIMUS solution for about 30 seconds again. Some staining of the pores took place indicating the penetration of EVEROLIMUS into the balloon wall membrane; however, the balloon burst at this pressure.

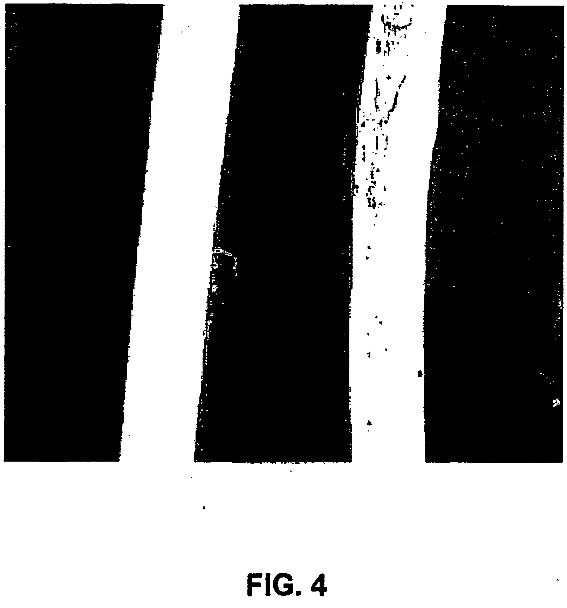

The balloon of the second sub-assembly was inflated using saline solution to about 6 atmospheres and immersed into the EVEROLIMUS solution for about 30 seconds again, followed by a visual inspection. The inspection revealed that the pores of the balloon were significantly stained by the blue dye, indicating substantial penetration of EVEROLIMUS into the wall of the balloon. Substantial penetration of EVEROLIMUS into the inflated balloon can be explained by the fact that the ePTFE balloon, in the inflated state, contained many pores, as shown in FIG. 3. The blue dye staining is also clearly shown in FIG. 4 which is an optical microphotograph in which an ePTFE balloon (left) is compared with a dyed ePTFE balloon (right).

The simulated experiment, therefore, has demonstrated that EVEROLIMUS can be loaded into the pores of the balloon when an inflated balloon is immersed into an EVEROLIMUS solution. The loading of EVEROLIMUS was not achieved when the balloon was uninflated.

### Example 2

To incorporate the drug into the balloon (e.g., made from ePTFE), the drug can be dissolved in a solvent (e.g., isopropyl alcohol, acetone, acetonitrile) to make a drug solution having a concentration of between about 0.1 mass % and about 30 mass %, such as between about 2 mass % and about 10 mass %, for example, about 5 mass %. The balloon can be then inflated using a fluid, such as saline solution, followed by immersing the inflated balloon in the drug solution for about 30 seconds. The inflation pressure can be between about 6 atmospheres and about 18 atmospheres, more narrowly, between about 12 atmospheres and about 18 atmospheres, for example, about 18 atmospheres (1 atmosphere = 101.3 kPa).

While particular embodiments of the present invention have been shown and described, it will be obvious to those skilled in the art that changes and modifications can be made without departing from this invention as defined by the appended claims.

## Claims

1. A method of modifying a balloon (10) of a catheter assembly (12), comprising:
(a) providing a balloon (10) comprising an inner layer made of a non-porous material and an outer layer made of a porous material;
(b)
(i) firstly inflating the balloon (10) from a collapsed configuration (14) to an inflated state; and secondly applying a substance to the balloon (10); or
(ii) firstly applying a substance to the balloon (10); and secondly inflating the balloon (10) from a collapsed configuration (14) to an inflated state,
in either alternative the substance being in a fluid form or carried by a fluid carrier and the inflated state of the balloon (10) being either maintained at the same or generally same level during the application of the substance to the balloon (10) or else increased or decreased, wherein the substance is deposited within the outer layer of the balloon (10); and
(c) contemporaneously with the application of the substance to the balloon (10), drying the balloon by blowing a gas at the balloon (10) with a flow speed of from about 300 feet/minute (91.5 meters/minute) to about 10,000 feet/minute (3047.85 meters/minute) for a duration of about 1 second to about 100 seconds, the gas having a temperature of from about 25ºC to about 200ºC,
wherein the substance and the gas are applied in a number of cycles until a desired amount of substance is retained by the balloon (10).

2. The method of claim 1, wherein the inflated state is greater than a range of an intended expanded configuration (16) of the balloon (10).

3. The method of claim 1, wherein the inflated state is less than a range of an intended expanded configuration (16) of the balloon (10).

4. The method of claim 1, wherein the balloon (10) has an intended expanded configuration (16) and the inflated state is a state in which the balloon (10) has a diameter above the intended expanded configuration (16) of the balloon (10) but less than a diameter which will cause the balloon (10) to be damaged or be no longer suitable for its intended use.

5. The method of claim 1, additionally including pulsating the balloon (10) to a greater and/or smaller size during the application of the substance to the balloon (10).

6. The method of claim 1, wherein the substance is one of or a combination of a therapeutic substance, a polymeric material and a blocking agent.

## Patentansprüche

1. Verfahren zur Modifizierung eines Ballons (10) einer Katheteranordnung (12), das Folgendes aufweist:
(a) Bereitstellen eines Ballons (10), der eine Innenschicht, die aus einem nichtporösen Material hergestellt ist, und eine Außenschicht aufweist, die aus einem porösen Material hergestellt ist;
(b)
(i) zunächst Aufblasen des Ballons (10) von einem zusammengefallenen Zustand (14) in einen aufgeblasenen Zustand; und anschließend Aufbringen einer Substanz auf den Ballon (10); oder
(ii) zunächst Aufbringen einer Substanz auf den Ballon (10); und anschließend Aufblasen des Ballons (10) von einem zusammengefallenen Zustand (14) in einen aufgeblasenen Zustand,
wobei in beiden Alternativen die Substanz eine Fluidform aufweist oder von einem Fluidträger getragen wird und der aufgeblasene Zustand des Ballons (10) entweder auf demselben oder im Allgemeinen demselben Niveau während des Aufbringens der Substanz auf den Ballon (10) gehalten wird oder erhöht oder verringert wird, wobei die Substanz innerhalb der Außenschicht des Ballons (10) abgeschieden wird; und
(c) zeitgleich mit dem Aufbringen der Substanz auf den Ballon (10), Trocknen des Ballons durch Blasen eines Gases auf den Ballon (10) mit einer Strömungsgeschwindigkeit von ungefähr 300 Fuß/Minute (91,5 Meter/Minute) bis ungefähr 10.000 Fuß/Minute (3047,85 Meter/Minute) über einen Zeitraum von ungefähr 1 Sekunde bis ungefähr 100 Sekunden, wobei das Gas eine Temperatur von ungefähr 25°C bis ungefähr 200°C aufweist,
wobei die Substanz und das Gas in einer Reihe von Zyklen aufgebracht werden, bis eine erwünschte Menge an Substanz von dem Ballon (10) gehalten wird.

2. Verfahren nach Anspruch 1, wobei der aufgeblasene Zustand größer als ein Umfang eines beabsichtigten erweiterten Zustands (16) des Ballons (10) ist.

3. Verfahren nach Anspruch 1, wobei der aufgeblasene Zustand geringer als ein Umfang eines beabsichtigten erweiterten Zustands (16) des Ballons (10) ist.

4. Verfahren nach Anspruch 1, wobei der Ballon (10) einen beabsichtigten erweiterten Zustand (16) aufweist und der aufgeblasene Zustand ein Zustand ist, in welchem der Ballon (10) einen Durchmesser aufweist, der größer als der beabsichtigte erweiterte Zustand (16) des Ballons (10), jedoch geringer als ein Durchmesser ist, der dazu führt, dass der Ballon (10) beschädigt wird oder nicht länger für seinen beabsichtigten Gebrauch geeignet ist.

5. Verfahren nach Anspruch 1, das zusätzlich ein Pulsieren des Ballons (10) in eine größere und/oder kleinere Größe während des Aufbringens der Substanz auf den Ballon (10) aufweist.

6. Verfahren nach Anspruch 1, wobei die Substanz eine der folgenden oder eine Kombination aus einer therapeutischen Substanz, einem Polymermaterial und einem Blockierungsmittel ist.

## Revendications

1. Procédé de modification d'un ballonnet (10) d'un ensemble de cathéter (12), comprenant le fait :
(a) de fournir un ballonnet (10) comprenant une couche interne réalisée en un matériau non poreux et une couche externe réalisée en un matériau poreux ;
(b)
(i) de gonfler, en premier lieu, le ballonnet (10) d'une configuration repliée (14) à un état gonflé ; et d'appliquer, en deuxième lieu, une substance au ballonnet (10) ; ou
(ii) d'appliquer, en premier lieu, une substance au ballonnet (10) ; et de gonfler, en deuxième lieu, le ballonnet (10) d'une configuration repliée (14) à un état gonflé,
Dans les deux variantes, la substance étant sous forme fluide ou portée par un support fluide et l'état gonflé du ballonnet (10) étant soit maintenu au même niveau ou globalement au même niveau au cours de l'application de la substance au ballonnet (10), ou augmenté ou diminué, où la substance est déposée dans la couche externe du ballonnet (10) ; et
(c) de sécher le ballonnet, simultanément avec l'application de la substance au ballonnet (10), en soufflant un gaz au ballonnet (10) avec une vitesse d'écoulement comprise entre environ 300 pieds/minute (91,5 mètres/minute) et environ 10000 pieds/minute (3047,85 mètres/minute) pendant une durée comprise entre environ 1 seconde et environ 100 secondes, le gaz ayant une température comprise entre environ 25°C et environ 200°C, où la substance et le gaz sont appliqués un certain nombre de cycles jusqu'à ce qu'une quantité souhaitée de la substance soit retenue par le ballonnet (10).

2. Procédé de la revendication 1, dans lequel l'état gonflé est supérieur à un intervalle d'une configuration expansée prévue (16) du ballonnet (10).

3. Procédé de la revendication 1, dans lequel l'état gonflé est inférieur à un intervalle d'une configuration expansée prévue (16) du ballonnet (10).

4. Procédé de la revendication 1, dans lequel le ballonnet (10) une configuration expansée prévue (16) et l'état gonflé est un état dans lequel le ballonnet (10) a un diamètre supérieur à la configuration expansée prévue (16) du ballonnet (10) mais inférieur à un diamètre qui amènera le ballonnet (10) à être endommagé ou à ne plus convenir à son usage prévu.

5. Procédé de la revendication 1, comportant en outre la pulsation du ballonnet (10) à une dimension supérieure et/ou inférieure au cours de l'application de la substance au ballonnet (10).

6. Procédé de la revendication 1, dans lequel la substance est l'une parmi une substance thérapeutique, un matériau polymère et un agent de blocage ou une combinaison de ceux-ci.
